# EUROPEAN PATENT APPLICATION

(11) **EP 1 634 584 A1**
(43) Date of publication of application: **15.03.2006**
(21) Application number: 03757632.9
(22) Date of filing: 08.10.2003
(51) Int. Cl.: A61K 9/08, A61K 47/30

(54) **DIMETICONE-CONTAINING SUSTAINED FORMULATION**

(30) Priority: 25.05.2003 CN 03138019; 16.06.2003 CN 03148883; 18.08.2003 CN 03153569; 11.09.2003 CN 03156876
(71) Applicant: Wang, Yuwan, 100094 Beijing (CN)
(72) Inventor: WANG, Yuwan, Haidian District Beijing 100094 (CN); PAN, Zhende; Beijing China Agriculture University, Road Haidian District Beijing 100094 (CN); DAI, Xiaoxi; Beijing China Agriculture University, Road Haidian District Beijing 100094 (CN)
(74) Representative: Wallis, Naomi Rachel
(86) International application number: PCT/CN2003/000849
(87) International publication number: WO 2004/103343

(57) **Abstract**

The present invention relates to preparation of sustained formulation by using dimeticone as carrier. Said formulation consists of active ingredient (e.g. drugs against parasites, insecticides, NSAIDs, antibiotics, sex hormone like agents or oily soluble vitamins) 0.5~40%(W/V), dimeticone 100% (V/V). Suitable stablizer, antioxidant, local analgesics and material for sustaining release also can be added. The formulation comprising abamectins agents against parasites, which is prepared with dimeticone as medium, can provide long-lasting action up to 50-120 days by subcutaneous injection in a single administration. The lasting period can be modified by adjusting the administration dosage. The formulation according to the present invention is bio-compatible, stable and injectable.

## Description

### Technical Scope

The present invention is a kind of sustained release formulation, characterised by the use of dimeticone as a medium to prepare the sustained formulation containing therapeutic drugs. These drugs include anti-parasitic drugs for animals, non-steroidal anti-inflammatory drugs (NSAIDs), sex hormone drugs and antibiotics. The formulation prepared with dimeticone as the medium is bio-compatible, stable and injectable, and such a formulation can be easily injected with an excellent sustained release effect.

### Background Technologies

1. Both Chinese patents with their respective application numbers of 03138019.0 and 03156876.9 have described the preparation of sustained release formulations containing anti-parasitic avermectin drugs with dimeticone as the medium; the Chinese patent with the application number of 03148883.8 has described the preparation of sustained release anti-parasitic formulations for animals containing N-phenyl pyrazolone (e.g. fipronil) drugs; the Chinese patent with application number of 03153569.0 has described the preparation of sustained release formulations containing NSAIDS with dimeticone as the medium. Recent research shows that formulations containing triazine anti-isospora drugs, formulations containing halofuginone anti-isospora drugs, formulations containing imidacloprid, formulations containing diflubenzuron, formulations containing ceftiofur, and formulations containing sex hormone drugs prepared with dimeticone as the medium, all have a satisfactory sustained release effect while causing a very low degree of damage to tissues at the injection site and being stable and injectable. Therefore, the present invention consists of the preparation of such sustained release formulations containing anti-isospora, sex hormone, or imidacloprid drugs with dimeticone as the medium in addition to the contents of Patents 03138019.0, 03156876.9, 03148883.8, and 03153569.0.
2. Dimeticone (abbreviated as silicon oil) is the general term for a series of polydimethyl siloxanes with a low molecular weight and different viscosity (Zheng Junmin et al (ed.), *Drug-use High Molecular Materials,* First edition, Medical Science Publishing House of China, August 2001, pp 169~170). It is a kind of highly hydrophobic oily liquid that tends to be extremely inert in terms of physiological activity, the viscosity of which varies very little within the range of the application temperature (-40~50°C). Dimeticone has excellent resistance to oxidation and can be sterilised at 150°C for an hour while being stable with most compounds, especially so with all drugs referred to in the present invention. All drugs and suspending agents (such as hydrogenated castor oil) referred to in the present invention hardly dissolve in dimeticone, and neither do they swell. All these characteristics form an important basis for dimeticone to be suitable for the preparation of formulations in the present invention.
   This test research shows that: (1) With 4~6ml dimeticone injected at each injection site subcutaneously on the body of an animal (cattle or sheep), there will be no obvious tissue damage to the injection site, no swelling, no agglomeration, and no side-effects such as granuloma. Therefore, concerning bio-compatibility, the preparation of formulations for subcutaneous injection can be prepared with dimeticone as the medium. (2) The solubility of such therapeutic drugs as anti-parasitic avermectin drugs, N-phenyl pyrazolone, anti-parasitic drugs, imidacloprid, diflubenzuron, triazine anti-isospora drugs , sex hormone drugs, and NSAIDs as well as ceftiofur, penicillin G potassium, or sodium in dimeticone is very low (less than 0.01%), thus the preparation of suspending agents of such therapeutic drugs with dimeticone as the medium is not problematic at all in terms of stability, which is one of the important factors affecting the sustained release effect of the formulation. (3) As hydrogenated castor oil neither dissolves nor swells in dimeticone, the liquid suspending agents prepared by using both dimeticone and hydrogenated castor oil jointly, with the content of hydrogenated castor oil being within an appropriate range (0.2~3.5%) will be better than suspending agents prepared with water, vegetable oil or other organic liquid as the medium in terms of stability, fluidity, injectability, and sustained release effect. Hydrogenated castor oil in such suspending agents plays a role to assist suspension and enhance the sustained release effect of the formulation, while in cases where micronisation and crystallisation is adopted to prepare formulations with the present invention, the existence of hydrogenated castor oil can prevent nucleuses of active ingredients from forming. It is therefore possible to prepare active particles with a fineness below 20 µm or even less. (4) Combine active ingredients with hydrogenated castor oil or hydrogenated castor oil/ ethyl cellulose to prepare the solid dispersion particles (with a fineness less than 300 µm), then disperse such particles in dimeticone and have the mixture ground with a colloid mill or a planetary mill; thus, sustained release formulations consisting of solid dispersion of various fineness (10~150 µm) can be prepared with the sustained release effect of the formulations adjusted by adjusting such factors as the fineness of the solid dispersions in the formulations and the proportions of hydrogenated castor oil and active ingredients. For example, combine avermectin drugs with hydrogenated castor oil to prepare solid dispersion particles with two respective proportions of 1:2 and 2:1 (avermectin drugs/hydrogenated castor oil). Then, scatter the solid dispersion of such two respective proportions into dimeticone according to a certain proportion, make the fineness of solid dispersions in the system fall within 85~120µm by controlling the degree of grinding and obtain sustained release formulations with sustained periods (effective periods) by adjusting the dosage. With one dose of such formulation, the effective period of such drugs can reach over 140 days at most and formulations thus prepared are better than sustained release formulations containing avermectin drugs/ hydrogenated castor oil solid dispersion particles prepared with vegetable oil, water or other liquids as the medium in terms of injectability, stability and bio-compatibility. While preparing sustained release formulations containing avermectin drugs/ hydrogenated castor oil solid dispersion particles with water as the medium, the first shortcoming is that it is difficult for the effective period to last more than 80 days. The second shortcoming is that due to the poor injectability, a "separation" between water and solid dispersions will occur upon injection without more water soluble (expansion) suspending agents added, thus making it impossible to complete the injection due to needle clogging. The third shortcoming is that phymas appear on the injection site while similar problems occur if other organic liquids are used as the medium. As a result, similar sustained injection formulations prepared in the past either failed to enter into a commercial phase or had the commercial value greatly diminished.
3. The avermectin drugs referred to in the present invention include abamectin, ivermectin, emamectin, eprinomectin, doramectin, moxidectin, 4"-deoxy-4"-methylamino-avermectin and other avermectin derivatives. A detailed description of avermectin anti-parasitic drugs or avermectin derivatives referred to in the present invention has been given in Patent CN 1345327A (WO00/58328). In terms of the chemical structure, all avermectin anti-parasitic drugs possess a macrolide structure, so they are also called macrolide anti-parasitic drugs. Such drugs can highly effectively drive away or kill nematodes and ectozoas such as mites, ticks, lice, and fly maggots which are extensively parasitic on the body of animals. With such drugs sold on the market dosed at 0.2~0.4mg per kilogramme of body weight, over 95% of parasites can be driven away and the effective period can last 10 to 20 days. High activity is an important biological basis for avermectin drugs to be appropriate for the preparation of sustained release formulations.
   Avermectin drugs are highly hydrophobic and hardly dissolve in dimeticone, which is the major chemical basis for avermectin drugs being able to be used to prepare the sustained formulations.
   Avermectin drugs cannot kill eggs but the life cycle for such parasites as sarcoptes scabiei and sucking lice to develop from eggs into imagoes is mostly about 20 days. Therefore, if common formulations or oral formulations are used to prevent and control such parasites, it is often the case that two or more dosages can make prevention and control effective. In addition, parasites like nematodes exist extensively in the environment and animals are liable to get infected repeatedly. A sustained effect of prevention and control can be achieved with one dose of sustained formulation. It is the existence of such problems that shows us that there lie broad prospects in the market to develop avermectin sustained formulations.
   In the formulations of the present invention, avermectin drugs can exist either in a state of fine particles (less than 100µm) or in a state of drug carrier particles. With the so-called drug carrier particles, it refers to solid dispersions or micro-spheres, micro-capsules, or liposomes or fine micro particles in a solid state, which are made up of both avermectin drugs and sustained release carrying materials. Sustained release carrying materials that can be combined into drug carrier micro particles together with avermectin drugs include hydrogenated castor oil, ethyl cellulose, polyesters, polyethylene glycol (PEG), and polyvinylpyrrolidone (PVP). More than one kind may be used together. The present invention has chosen hydrogenated castor oil, hydrogenated castor oil/ethyl cellulose, hydrogenated castor oil/ethyl cellulose/PVP, ethyl cellulose/PVP, hydrogenated castor oil/PVP, hydrogenated castor oil/ethyl cellulose/PEG, hydrogenated castor oil/PEG or ethyl cellulose/PEG to combine with avermectin drugs into solid dispersions. In addition, the quick release rate and the sustained release effect of formulations can be adjusted by changing the proportions of water-soluble sustained release carriers (such as PVP and PEG) and hydrophobic sustained release carriers in solid dispersions (such as hydrogenated castor oil and ethyl cellulose). All sustained release carrier materials chosen for the present invention do not dissolve or swell in dimeticone, which is an important basis to ensure the stability of drug carrier micro particles in the formulation and to determine the sustained release effect of formulations.
   Experiments show that it is very hard to grind avermectin drugs to an ideal fineness with airflow pulverisation and it is very hard to meet the requirement of aseptic manipulation, as there is a large consumption of energy and dust pollution. So the present invention prefers to prepare the ultra-micro powder of avermectin drugs with the method of micronisation and crystallisation as the first priority. And the principles of this method are as follows: dissolve and melt avermectin drugs and hydrogenated castor oil into a kind of organic solvent with a low boiling point (such as ethanol, ethyl acetate or chloroform) being heated and then have the solvent cooled down and add in dimeticone under agitation. Avermectin drugs can thus be separated out in the state of micro crystallisation. Alternatively, scatter avermectin drugs into a small quantity of dimeticone to produce a viscous and dense liquid and then have the liquid wet grinded (for example, to conduct the grinding with a planetary mill or a colloid mill), then add the residual medium to the final volume. Avermectin drugs and carrier materials like hydrogenated castor oil can also be combined together to prepare solid dispersions, ground into particles and scattered into dimeticone so as to prepare formulations of the present invention.
   The sustained formulations containing avermectin drugs prepared according to the present invention can be administered by an injection with a dosage of 1~3 mg avermectin drugs/ kg b.w. After one dosage, the drugs' effective period can last 60~120 days or even longer and the sustaining time of the drugs' effect can be changed by changing the dosage.
4. N-phenyl pyrazolone drugs referred to in the present invention form a group of chemically synthesised drugs that can play a strong role in killing insects, with fipronil as a typical example. Fipronil has been extensively used in the prevention and control of agricultural pests, and it is mainly used to drive away and kill such ectozoas as fleas and ticks so far as the prevention and control of animal verminoses is concerned. In Patent CN1018733B, there are detailed descriptions of the preparation of fipronil and the preparation and application of such formulations. Products containing fipronil that have been sold in the market include diadermic pour-on solutions and spray solutions, which are mainly used to drive away and kill fleas on the body of cats or dogs or ticks on the body of cattle.
   The present invention is to have fipronil prepared into sustained formulations with dimeticone and hydrogenated castor oil as the medium. Such formulations are used for the prevention and control of fleas and ticks on the body of cattle. With a dosage of this formulation by way of subcutaneous injection, the effect of the medicine can last for 3-6 months or even longer.
5. Non-steroidal anti-inflammatory drugs (NSAIDs) referred to in the present invention can play a role in allaying fever, easing pain and also have an anti-inflammatory action, just as introduced in detail in some pharmic works and pharmaceutical manuals (Zhang Wensheng and Li Anliang, et al (ed.), *Pharmaceutic Chemistry,* Higher Education Press, 1999, pp348~375). NSAIDs include the category of fever allaying drugs and pain easing drugs and the category of anti-inflammatory drugs, such as salicylic acids, pyrazolones, arylkanoic acids, fenamic acids, benzothiazines, etc.; all these anti-inflammatory drugs can bring their effect into play by inhibiting cyclooxygenase (COX) so as to disturb the biological synthesis of prostaglandin. Thus, the present invention also includes other water insoluble COX-2 inhibitors. Included in the present invention are NSAIDs that are water insoluble or water micro-soluble or those that can be converted to be water insoluble after a certain chemical reaction. Preferred NSAIDs to be used for the preparation of formulations of the present invention include indomethacin, ketoprofen, meloxican, naproxen, caprofen, ketorolac, flunixin, diclofenac, piroxican, etc. The present invention uses dimeticone and hydrogenated castor oil as the sustained release carrier material to prepare sustained release formulations containing NSAIDs, which are suitable for subcutaneous injection on the body of animals as they have such advantages as good stability, being bio-compatible, and injectable with a good sustained release effect.
6. Imidacloprid referred to in the present invention has been extensively used in the prevention and control of agricultural pests and it is mainly used on the body of animals for the prevention and control of such ectozoas as fleas and lice on the body of cats and dogs. Products already being sold in the market are diadermic pour-on preparations, a 10mg/kg b.w dosage of which can remain effective for approximately one month. The sustained release formulations of the present invention can be used for the prevention and control of ectozoas on the body of pets, and a 10~100mg/kg b.w dosage by way of subcutaneous injection can remain effective for 80-360 days. Diflubenzuron referred to in the present invention is a kind of benzoyl-phenylthiourea pesticide, which can inhibit insects' synthesis of chitin, making it impossible for larvae to form a new cuticle in ecdysis so that larvae will die of the deformity. Diflubenzuron has been extensively used for the prevention and control of agricultural pests, and it has been prepared into liquid formulations to be used in a sheep dip to drive away and kill lice. The present invention uses dimeticone as the medium to prepare diflubenzuron into formulations to drive away and kill such ectozoas as fleas and lice by subcutaneous injection. The effectiveness of a single dosage can last for 2-4 months. Triflumuron, cyromazine, oestrogen, progesterone, androgens, triazine anti-isospora drugs (like toltrazuril and diclazuril), cephalosporin antibiotics (such as ceftiofur hydrochloride, sodium salt or free acid) and other antibiotic drugs are already known and are sold on the market. The present invention is to combine them with dimeticone or dimeticone/hydrogenated castor oil or dimeticone/hydrogenated castor oil/ ethyl cellulose to prepare sustained release formulations.

### Contents of Invention

The present invention is to prepare sustained formulations with dimeticone as the dispersion medium, and the said formulation consists of:
a) Therapeutic drugs or active ingredients 0.5~40% (W/V);
b) Dimeticone up to100% (V/V); and
c) Other suitable auxiliary agents, such as non-ionic surfactants, suspending agents, materials for sustained release, antioxidants and local analgesics.

The said therapeutic drugs or active ingredients are as follows:
(1) Avermectin anti-parasitic agents, including abamectin, ivermectin, emamectin, eprinomectin, doramectin, moxidectin, 4"-deoxy-4"-metylamino-avermectin B₁ and other avermectin derivatives. For a detailed description of avermectin anti-parasitic agents, see Patent WO00/58328.
(2) Non-steroidal anti-inflammatory drugs (NSAIDs), including salicylic derivatives, pyrazolones, para-amino phenol derivatives, indole and indene acetic acid, arylkanoic acids (such as heteroaryl acetic acid and aryl propionic acid), 1,2-benzothiazine (such as enolic acids), alkanone, anthranalic acids (fenamic acids) and other COX-2 inhibitors. The preferred non-steroidal anti-inflammatory drugs used in the preparation of the formulation of the present invention are indomethacin, ketoprofen, meloxican, naproxen, caprofen, ketorolac, flunixin, diclofenac and piroxican etc.
(3) Other anti-parasitic agents, including imidacloprid, diflubenzuron, lufenuron, methoprene, fipronil, pyridinol, cyromazine, triazine anti-isospora drugs (toltrazuril, diclazuril), albendazole sulphoxide or albendazole sulphoxide hydrochloride, closantel or its sodium salt.
(4) Antibiotic agents, including cephalosporins, cillins, ESBL inhibitors, thiamulin base or thiamulin fumerate, tylosins (such as tylosin, tilmicosin, isvaleryl tylosin tartrate), doxycycline or doxycycline hydrochloride, minocycline, gentamycin, lincomycin, clindamycin, neomycin, polymycin, quinolone antibiotics and sulphanilamide antibiotics. More than one of these can be used in combination in a formulation.
(5) Sex hormone agents including oestrogen, progesterone and androgen.
(6) Oily soluble vitamins.
(7) Water-insoluble or water micro-soluble mineral elements.

The said non-ionic surfactants, suspending agents and materials for sustained release include glycerine fatty acid esters, polyglycerol fatty acid esters, sucrose esters, sorbitan fatty acid esters (Span), polyoxyethylene sorbitan fatty acid ester condensate (Tween), polyoxyethylene fatty acid condensate (Myrjs), polyoxyethylene fatty alcohol condensate (Brijs), Peregal, emulsifier (OP), polyoxyethylene castor oil condensate, polyoxyethylene hydrogenated castor oil condensate, Pluronic, fatty acid esters which are in solid state below 40° C (such as glycerine monostearate, hydrogenated castor oil and Carnauba Wax), lanolin, cetyl alcohol, stearic acid, polyvinylpyrrolidone (PVP), polyethylene glycol with a molecular weight greater than 1,000, gelatine, Arabic gum, ethyl cellulose and polyvinyl butyral. The especially preferred stabilisers and materials for sustained release are Tween, Span, ethyl cellulose, hydrogenated castor oil, aluminium stearate, polyvinylpyrrolidone (PVP) and polyethylene glycol with a molecular weight greater than 1,000. Two or more of these may be used in combination.

The said antioxidants are oily-soluble ones with a content of 0.01-0.5% (WN) in the formulation. Such preferred antioxidants as dibutyl hydroxytoluene (BHT), propyl gallate (PG), and tertiary butyl para-hydroxy anisole (BHA) can be used in combination with an application amount of 0.01-0.5% (WN).

The said local analgesics include trichloro-tert-butyl-alcohol, benzyl alcohol, procaine, tetracaine and lidocaine.

The composition of formulations containing avermectin drugs of the present invention is:
a) Avermectin drug 0.5~30% (WN); b) Dimeticone up to 100% (VN); c) Other auxiliary agents such as stabilisers, antioxidants and local analgesics may be added.

The composition of preferred formulations is: a) Avermectin drugs 1-10% (WN); b) Hydrogenated castor oil 0-10% (WN); c) Dimeticone up to 100% with a viscosity of less than 100mm²/S (VN) d) Antioxidants and local analgesics may also be added.

### The preferred preparation methods are as follows:

Method (1): Take some avermectin agent and add alcohol, acetone or another organic solvent with a low boiling point, making the amount of the organic solvent 2~5 times that of the avermectin drug, with or without hydrogenated castor oil added. The avermectin drug is dissolved/melted by raising the temperature to approximately 85°C. When the mixture is cooled down under agitation, add dimeticone up to the final volume. Remove alcohol, acetone or another organic solvent with a low boiling point by depressurising the mixture and homogenise, (for example, through a colloid mill).
Method (2): Add a small amount of dimeticone to the avermectin drug and hydrogenated castor oil, and then melt the hydrogenated castor oil at approximately 90°C. When the mixture has cooled down under agitation into a fatty or relatively viscous and dense liquid, grind (for example, with a colloid mill or a planetary mill). When the fineness of the avermectin drug has reached below 100 µm, add the residual medium to the final volume.
Method (3): Scatter the micronised avermectin drug (with a fineness of less than 100 µm) into hydrogenated castor oil that has already been melted. Then add dimeticone, homogenise, and add the residual medium to the final volume.
Method (4): Scatter the solid dispersion containing the avermectin drugs and hydrogenated castor oil into dimeticone. Heat the mixture to approximately 90°C under agitation, then have it cooled down and homogenised under agitation. Add the residual medium and auxiliary agents to the final volume.
Method (5): Scatter the solid dispersion containing the avermectin drugs and hydrogenated castor oil into dimeticone, and then have the mixture ground (for example, with a colloid mill or a planetary mill) and homogenised. When the fineness of the solid dispersion reaches below 120 µm, add the dimeticone and auxiliary agent up to the final volume.

The preferred composition of formulations containing the avermectin drugs and the preparation method are as follows:
(1) Composition of formulations:
   a) Drug carrier particles containing avermectin drugs 2-35% (WN);
   b) Suspending agents (for example, hydrogenated castor oil) 0-3%(WN);
   c) Local analgesic 0.5-2.5% (WN);
   d) Dimeticone up to 100% (V/V).
(2) Preparation method:

Scatter drug carrier particles containing avermectin drugs with a fineness of less than 360µm, then have the mixture ground (for example, with a colloid mill or a planetary mill), add dimeticone with or without suspending agents to the final volume when the drug carrier particles are less than 150µm. After being mixed thoroughly, the product is obtained. Or alternatively, scatter drug carrier particles containing avermectin drugs with a fineness of less than 120µm into dimeticone with or without suspending agents, and homogenise.

The said drug carrier particles containing avermectin drugs are solid dispersion with the combination of avermectin drugs anti-parasitic drugs and sustained release carrier materials or drug carrier particles of other types (such as microspheres, micro-capsules or nano-particles).

The said sustained release carrier materials consist of hydrophilic and hydrophobic carrier materials. The preferred hydrophilic carrier materials are gelatine, Arabic gum, polyethylene glycol with a molecular weight greater than 1,000, and polyvinylpyrrolidone (PVP). The preferred hydrophobic carrier materials are ethyl cellulose, polyvinyl butyral and hydrogenated vegetable oil (for example, hydrogenated castor oil). The especially preferred carrier materials are ethyl cellulose, hydrogenated castor oil, polyethylene glycol with a molecular weight greater than 1,000 and polyvinylpyrrolidone (PVP), more than one of which can be used in combination.

The sustained release formulations containing avermectin drugs of the present invention can be used for the prevention and treatment of parasitic diseases in animals. The preferred administration is subcutaneous injection, especially subcutaneous injection behind the ear, in the neck or in the back of the animals. The administration dosage is 0.2~6mg of active ingredient per kilogram of body weight (kg b.w), and the appropriate dosage is 1~3mg of active ingredient per kilogram of body weight. The effective period of each administration is 40~140 days, depending on the dosage. Therefore, veterinarians or technicians in this field can adjust the dosage according to the requirement to meet the required effective period. This formulation will cause neither swelling of the injection site nor any degree of damage to the tissues.

The composition of formulations containing non-steroidal anti-inflammatory drugs of the present invention is:
a) Non-steroidal anti-inflammatory drugs 1-15% (W/V);
b) Hydrogenated castor oil 0-5% (W/V);
c) Dimeticone up to 100% (V/V);
d) Antioxidants and local analgesics may be added.

### The preparation methods are as follows:

Method (1): Take some non-steroidal anti-inflammatory drugs and add alcohol, acetone or another organic solvent with a low boiling point, making the amount of the organic solvent 2~5 times that of the non-steroidal anti-inflammatory drug, with or without hydrogenated castor oil added. The non-steroidal anti-inflammatory drug is dissolved/melted by raising the temperature to approximately 85°C. When the mixture is cooled down under agitation, add dimeticone in to the final volume. Remove alcohol, acetone or another organic solvent with a low boiling point by depressurising the mixture, then homogenise the mixture (for example, through a colloid mill).
Method (2): Add a small amount of dimeticone to the non-steroidal anti-inflammatory drug and hydrogenated castor oil, and then melt the hydrogenated castor oil at approximately 90°C. When the mixture has cooled down into a fatty or relatively viscous and dense liquid under agitation, grind (for example, with a colloid mill or a planetary mill). When the fineness of the non-steroidal anti-inflammatory drug has reached below 100 µm, add the residual medium and auxiliary agent to the final volume.
Method (3): Scatter the micronised non-steroidal anti-inflammatory drug (with a fmeness of less than 100 µm) into hydrogenated castor oil that has already been melted. Then add dimeticone, homogenise the mixture and add the residual medium and auxiliary agent to the final volume.
Method (4): Scatter solid dispersion containing non-steroidal anti-inflammatory drugs and hydrogenated castor oil into dimeticone. Heat the mixture to approximately 90°C under agitation, then have it cooled down and homogenised under agitation. Add the residual medium and auxiliary agent to the final volume.
Method (5): Scatter solid dispersion containing the non-steroidal anti-inflammatory drugs and hydrogenated castor oil into dimeticone, and then grind the mixture (for example, with a colloid mill or a planetary mill) and homogenise. When the fineness of the solid dispersion reaches below 120 µm, add the dimeticone and auxiliary agent up to the final volume.

The composition and preparation methods of sustained release formulations containing fipronil or diflubenzuron or imidacloprid of the present invention are as follows:

### (1) Composition of formulations:

a. Fipronil, diflubenzuron or imidacloprid 2~10% (W/V);
b. Hydrogenated castor oil 0.2~5% (WN);
c. Dimeticone up to 100% (V/V);
d. Antioxidants and local analgesics may be added.

### (2) Preparation methods

Method (a): Take some fipronil or imidacloprid and add alcohol or acetone or other organic solvent with a low boiling point, dissolve/melt the mixture by raising the temperature to approximately 85°C with hydrogenated castor oil added. When the mixture is cooled down under agitation, add dimeticone and auxiliary agent up to the final volume. Remove alcohol, acetone or another organic solvent with a low boiling point by depressurising the mixture, then homogenise the mixture (for example, through a colloid mill).
Method (b): Add a small amount of dimeticone to fipronil, diflubenzuron or imidacloprid and hydrogenated castor oil, and then melt hydrogenated castor oil at approximately 90°C. When the mixture has cooled down under agitation into a fatty or relatively viscous and dense liquid, grind the liquid (for example, with a colloid mill or a planetary mill). When the fineness of fipronil, diflubenzuron or imidacloprid has reached below 100 µm, add the residual medium and auxiliary agent up to the final volume, thus obtaining the product.
Method (c): Scatter the micronised fipronil, diflubenzuron or imidacloprid (with a fineness of less than 100 µm) into hydrogenated castor oil that has already been melted. Then add dimeticone, homogenise the mixture and add the residual medium and auxiliary agent to the final volume.
Method (d): Scatter solid dispersion containing fipronil, diflubenzuron or imidacloprid and hydrogenated castor oil into dimeticone. Heat the mixture under agitation to approximately 90°C, then have it cooled down and homogenised while further agitating when the mixture has melted. Add the residual medium and auxiliary agent to the final volume.
Method (e): Scatter solid dispersion containing fipronil, diflubenzuron or imidacloprid and hydrogenated castor oil into dimeticone, and then grind the mixture (for example, with a colloid mill or a planetary mill) and homogenise. When the fineness of the solid dispersion reaches below 120 µm, add the dimeticone and auxiliary agent up to the final volume.

The composition and preparation methods of sustained release formulations containing penicillin or cephalosporin drugs of the present invention are as follows:

### (1) Composition of formulations:

a. Penicillins or cephalosporin drugs 2-40% (WN);
b. Hydrogenated castor oil 0-5% (WN);
c. Dimeticone up to 100% (V/V);
d. Antioxidants and local analgesics may be added.

### (2) Preparation methods

Method (a): Add a small amount of dimeticone to the penicillin or cephalosporin drugs to make them a dense and viscous liquid, and then either grind the liquid (for example, with a colloid mill or a planetary mill) or not. Add in dimeticone containing hydrogenated castor oil in a smelted state under agitation, have the mixture cooled down into a fatty or a dense and viscous liquid and grind (for example, with a colloid mill or a planetary mill). When the fineness of the penicillin or cephalosporin drugs has fallen below 100µm, add in the residual medium and auxiliary agent, thus obtaining the product.
Method (b): Scatter the micronised penicillins or cephalosporin drugs (with a fineness of less than 100 µm) into hydrogenated castor oil that has already been melted. Then add dimeticone, homogenise the mixture and add the residual medium and auxiliary agent to the final volume.
Method (c): Add a small amount of dimeticone to penicillin or cephalosporin drugs to make them a viscous liquid, grind the mixture (for example, with a colloid mill or a planetary mill) until the fineness of the penicillin or cephalosporin drugs reaches below 100 µm, then add in the residual medium and auxiliary agent to the final volume, thus obtaining the product.

All other therapeutic drugs referred to in the present invention can be prepared into a sustained release formulation with dimeticone or dimeticone/hydrogenated castor oil or dimeticone/hydrogenated castor oil/ethyl cellulose as a medium. Technicians in this field can easily master the preparation or application techniques.

### Specific Methods of Implementation

The following examples of implementation are provided to enable the present invention to be thoroughly understood, but such examples cannot be interpreted as restrictions on the scope of claims and the core content of the present invention.

### Example 1 Preparation of suspension formulation containing 10% abamectin

Take 1 kg of abamectin, add 3~4 litres of ethanol and dissolve at 90°C. Cool the mixture under agitation and add 5 litres of dimeticone with a viscosity of 100 mm²/S under agitation. Remove the ethanol by reducing the pressure. Grind the mixture with a colloid mill, add dimeticone containing trichloro-tert-butyl-alcohol and antioxidants constituting 0.5% of the total volume up to the final volume. The formulation is then prepared.

### Example 2 Preparation of suspension formulation containing 5% doramectin

Take 1 kg of doramectin and 0.3 kg of hydrogenated castor oil, add 5 litres of dimeticone with a viscosity of 20 mm²/S and dissolve the mixture at 90°C. Then cool the mixture under agitation into a fatty liquid. Grind to the fineness of doramectin below 100 µm with a colloid mill, then add dimeticone containing trichloro-tert-butyl-alcohol and antioxidant to the final volume. The formulation is then prepared.

### Example 3 Preparation of suspension formulation containing 20% ivermectin

Take 2 kg of ivermectin and 0.2 kg of hydrogenated castor oil and add 6 litres of ethanol to dissolve at 90°C. Then cool the mixture under agitation, while adding in 8 litres of cold dimeticone with a viscosity of 20 mm²/S. Continue agitating and remove ethanol by reducing the pressure. With the mixture ground with a colloid mill, the fatty suspension formulation is then prepared.

This formulation is used for the prevention and control of parasitic diseases in cattle and sheep. The formulation is injected by subcutaneous means behind the ear or on the neck with a dosage of 2-3 mg per kg b.w. The effect can last for more than 120 days after a single injection (implant). This formulation can be administrated (implanted) by subcutaneous injection with a normal injector (with a needle of #16 or above) without operation. It is therefore preferable to a solid implant.

### Example 4 Preparation of sustained formulation containing 5% acetamino abamectin

Take 14 kg of solid dispersion particles containing 71.4% acetamino abamectin and 28.6% hydrogenated castor oil (W/W), add 90 litres of dimeticone, and then homogenise. When the mixture has been ground to a fineness of dispersion below 100 µm with a colloid mill, add 100 litres of dimeticone containing trichloro-tert-butyl-alcohol and antioxidant, and homogenise. The formulation is then prepared.

### Example 5 Preparation of suspension formulation containing 5% ivermectin

Take 1 kg of ivermectin and 0.3 kg of hydrogenated castor oil, add in 3 litres of ethanol and dissolve at 90°C. Then cool the mixture under agitation and add in 9 litres of dimeticone with a viscosity of 20 mm²/S when the mixture has solidified. Agitate again and remove the ethanol by reducing pressure. With the mixture ground with a colloid mill, add in dimeticone containing trichloro-tert-butyl-alcohol and antioxidant to the final volume. The formulation is then prepared.

### Example 6 A plasma concentration analysis of the formulation in Example 5

With sheep as the test animal, inject the formulation in Example 5 subcutaneously with a dosage of 1.5 ml per 50 kg b.w. Collect plasma samples from the test sheep at a regular time to determine the concentration of ivermectin in its plasma using the fluorescence - high pressure liquid chromatography method. The results are as follow:

| | | | | | | | | | | | |
|---|---|---|---|---|---|---|---|---|---|---|---|
| Sampling time (days) | 1 | 2 | 3 | 5 | 7 | 10 | 35 | 50 | 65 | 80 | 95 |
| Plasma concentration (ng/ml) | 7 | 12 | 16 | 18 | 25 | 17 | 11 | 6 | 4 | 2 | 1.1 |

### Example 7 Preparation of a sustained formulation containing 6% ivermectin

Take 9 kg of solid dispersion particles (with a fineness below 300 µm) of ivermectin/hydrogenated castor oil (1:2, W/W) and 4.5 kg of solid dispersion particles (with a fineness below 300 µm) of ivermectin/hydrogenated castor oil (2:1, W/W), and add in 30 to 50 litres of dimeticone with a viscosity of 20 mm²/S. When the mixture has been ground to a fineness of dispersion between 90 and 120 µm with a colloid mill or a planetary mill, add dimeticone (20 mm²/S) containing trichloro-tert-butyl-alcohol and antioxidant up to the final volume (100 litres).

### Example 8 A comparison conducted on the plasma concentration analysis for formulation in Example 7 and 6% water suspension of ivermectin

Take sheep as the test animals, and divide them into 3 groups with 5 sheep in each group. For Group 1, inject the formulation in Example 7 with a dosage of 1.5 ml per 50 kg b.w. (1.8 mg ivermectin/kg b.w.) subcutaneously. For Group 2, inject subcutaneously 1.5 ml 6% water suspension of ivermectin (6% ivermectin, 20% 1,2- propylene glycol, 8% polyethylene glycol 10000, add water to 100%) (1.8 mg ivermectin/kg b.w.). For group 3, inject 1.5 ml of 6% water suspension of ivermectin (solid dispersion particles of 12% ivermectin/hydrogenated castor oil (1:1), 8% polyethylene glycol 10000, add water to 100%) subcutaneously. Collect the plasma sample at a regular time from the test sheep so as to determine the concentration of ivermectin in the plasma. The results are as follow (the figures are an average value):

| Sampling time (days) | 1 | 3 | 5 | 7 | 10 | 35 | 50 | 65 | 80 | 100 | 120 |
|---|---|---|---|---|---|---|---|---|---|---|---|
| Group 1 | 5 | 11 | 14 | 22 | 22 | 15 | 9 | 8 | 6 | 4 | 4 |
| Group 2 | 16 | 35 | 28 | 19 | 11 | 6.7 | 3.8 | 2.3 | 0.6 | 0 | 0 |
| Group 3 | 14 | 31 | 26 | 17 | 10 | 5.1 | 4.4 | 3 | 1.2 | 0.3 | - |

### Example 9 Observation on the levels of damage to tissues at the injection site caused by the formulation of Group 1, Group 2 and Group 3 in Example 8 respectively

Choose cattle with a weight of 180~200 kg as test animals. Divide them into 3 groups with 10 cattle in each group. Each group of cattle will be injected with one of the groups of formulations described in Example 8. Observe for swelling or agglomeration at the sites of injection during 1 to 20 days after injection. The results are as follows: no swelling or agglomeration were observed at the sites of injection for Group 1 and Group 2 between 1 and 20 days, but 3 to 5 cm swellings were found at the sites of injection on 4 cattle in Group 3 between 2 and 14 days after the injection, and such swellings did not disappear until 20 days.

### Example 10 Variation of plasma concentration caused by variant dosage of formulation in Example 7 administrated to sheep

Take sheep as the test animals. Divide them into 3 groups with 5 sheep in each group. For Group 1, inject the formulation in Example 7 at a dosage of 1 ml/50 kg b.w. (1.2 mg ivermectin/kg b.w.). For Group 2, inject the formulation in Example 7 at a dosage of 1.5 ml/50 kg b.w. (1.8 mg ivermectin/kg b.w.). Collect plasma samples at different times to determine the concentration of ivermectin in the plasma (ng/ml). The results are as follow (the figures are an average value):

| Sampling time (days) | 3 | 5 | 7 | 10 | 30 | 50 | 70 | 90 | 110 |
|---|---|---|---|---|---|---|---|---|---|
| Group 1 | 6 | 11 | 16 | 21 | 14 | 6 | 4 | 1.5 | 0.6 |
| Group 2 | 7 | 16 | 18 | 24 | 17 | 11 | 7 | 4 | 3 |

### Example 11 Sustained formulation comprising 7% fipronil

(1) Composition of formulation: a) Fipronil 7% (WN); b) Hydrogenated castor oil 1.5% (WN); c) Trichloro-tert-butyl-alcohol 0.5% (WN); d) Dimeticone (20 m²/S) 100% (V/V).
(2) Preparation method: mix fipronil with acetone at the ratio of 1:1 and heat the mixture at 80°C until it is completely dissolved. Then mix with melted hydrogenated castor oil. When the mixture becomes semi-solidified by stirring it up with a magnetic stirrer under normal temperature, add dimeticone (20 mm²/S) of a quantity which is 3 to 4 times that of fipronil, and agitate continuously while reducing pressure. When acetone has been volatilised completely, grind the mixture with a colloid mill, and add in trichloro-tert-butyl-alcohol and the residual dimeticone to the final volume.

### Example 12 Sustained formulations containing solid dispersion particles of fipronil/ hydrogenated castor oil

(1) Composition of formulation: a) Solid dispersion particles of fipronil/ hydrogenated castor oil (1:0.5) 15% (WN); b) Dimeticone (20 mm²/S) to 100% (VN).
(2) Preparation method: a) Preparation of solid dispersion particles: mix fipronil with acetone at 1:1 to 1:1.2 (W/V) and heat until completely dissolved. Then mix with melted hydrogenated castor oil. When the mixture has solidified through agitation, remove the acetone by drying or reducing pressure, and obtain a solid dispersion containing fipronil/ hydrogenated castor oil. Grind the solid dispersion mechanically to a fineness below 300 µm, then obtain the solid dispersion particle containing fipronil/ hydrogenated castor oil. b) Preparation of formulation: Scatter the said solid dispersion particles into dimeticone of a certain quantity, and grind with a colloid mill to a fineness between 90 to 120 µm, and then add the residual dimeticone to the final volume. The formulation is then prepared.

### Example 13 Sustained formulation containing fipronil particles

(1) Composition of formulation: a) Fipronil particles 10% (WN); b) Hydrogenated castor oil 1.5% (WN); c) Dimeticone (20 mm²/S) up to 100% (VN).
(2) Preparation method: Scatter micronised fipronil (with a fineness below 75 µm) into dimeticone with a volume equivalent to approximately 50% of the final volume of the formulation. After stirring it evenly, add the residual dimeticone containing hydrogenated castor oil to the final volume. Grind with a colloid mill and homogenise.

### Example 14 Sustained formulation containing ketoprofen

(1) Composition of formulation: a) Ketoprofen 10% (W/V); b) Hydrogenated castor oil 1.5% (WN); c) Trichloro-tert-butyl-alcoho10.5% (WN); d) Dimeticone (20 mm²/S) up to 100% (VN).
(2) Preparation method: mix fipronil with ethanol at a ratio of 1:1 and heat the mixture at 40°C until completely dissolved, then mix evenly with melted hydrogenated castor oil. When the mixture has become semi-solidified through agitation with a magnetic stirrer under normal temperature, add dimeticone (20 mm²/S) of a quantity 3 to 4 times that of the ketoprofen, and agitate continuously while reducing pressure. When the ethanol has volatilised completely, grind with a colloid mill, and add trichloro-tert-butyl-alcohol and the residual dimeticone to the final volume.

### Example 15 Sustained formulation containing diclofenac particles

(1) Composition of formulation: a) Diclofenac particles 10% (WN); b) Hydrogenated castor oil 2% (WN); c) Dimeticone (20 mm²/S) up to 100% (V/V).
(2) Preparation method: Scatter micronised diclofenac (with a fineness below 75 µm) into dimeticone of which the volume is equivalent to approximately 50% of the final volume of the formulation. When it has been agitated evenly, add the residual dimeticone containing hydrogenated castor oil up to the final volume. Grind with a colloid mill and homogenise.

### Example 16 Sustained formulation containing ceftiofur hydrochloride particles

(1) Composition of formulation: a) Ceftiofur hydrochloride particles 15% (WN); b) Hydrogenated castor oil 2% (WN); c) Trichloro-tert-butyl-alcohol 0.5% (W/V); d) Dimeticone (20 mm²/S) up to 100% (V/V).
(2) Preparation method: Scatter micronised ceftiofur hydrochloride (with a fineness below 100 µm) into dimeticone of which the volume is equivalent to approximately 50% of the final volume of the formulation. When it has been agitated evenly, add the residual dimeticone containing melted hydrogenated castor oil and trichloro-tert-butyl-alcohol up to the final volume. Grind with a colloid mill and homogenise

### Example 17 Liquid formulation containing penicillin G potassium

(1) Composition of formulation: a) Penicillin G potassium35% (WN); b) Hydrogenated castor oil 0.5% (WN); c) Trichloro-tert-butyl-alcohol 0.5% (WN); d) Dimeticone (20 mm²/S) up to 100% (V/V).
(2) Preparation method: Scatter penicillin G potassium into dimeticone of which the volume is equivalent to approximately 50% of the final volume of the formulation. When it has been agitated evenly, grind the penicillin G potassium to a fineness less than 150 µm with a colloid mill, then add in dimeticone containing melted hydrogenated castor oil and trichloro-tert-butyl-alcohol up to the final volume. Grind with a colloid mill and homogenise.

### Example 18 Preparation of sustained formulation containing 6% ivermectin

Take 11 kg of ivermectin, add 4 kg of melted hydrogenated castor oil and 18 to 22 litres of ethyl acetate. Dissolve the ivermectin by heating or refluxing the mixture, and cool it rapidly. When the mixture has solidified, remove the ethyl acetate by reducing pressure, dry continuously and then add in dimeticone containing 1 kg of trichloro-tert-butyl-alcohol and 0.2 kg BHT to the volume of 200 litres. Agitate the mixture and heat it to 90°C to melt the hydrogenated castor oil, then cool the mixture to below 30°C under agitation, and the formulation will be obtained after homogenisation (with a colloid mill or a 100 mu sieve).

### Example 19 Preparation of sustained formulation containing 14% albendazole sulphoxide hydrochloride

Take 14g of albendazole sulphoxide hydrochloride and 1g of hydrogenated castor oil and add 40ml dimeticone. Melt the hydrogenated castor oil at 90°C, and then cool it to below 30°C under agitation. Add in dimeticone to the final volume and the formulation will be obtained after homogenisation.

### Example 20 Preparation of sustained formulation containing 10% closantel sodium

Scatter 13g of solid dispersion closantel sodium/hydrogenated castor oil particles (10:3, W/W) into about 40ml of dimeticone (20 mm²/S), and grind so that the fineness of the solid dispersion particles of closantel sodium/hydrogenated castor oil falls between 80 and 130 µm, then add in dimeticone containing antioxidant up to the final volume. Homogenise.

### Example 21 Preparation of sustained formulation containing 4% doramectin

Take 9kg of solid dispersion particles (with a fineness below 300µm) of doramectin/ hydrogenated castor oil/ethyl cellulose (1:0.5:0.3, W/W/W), add in 30-50ml of dimeticone (20 mm²/S), and grind with a colloid mill or a planetary mill so that the fineness of solid dispersion in the system falls between 70µm and 100 µm, then add in dimeticone (20 mm²/S) containing trichloro-tert-butyl-alcohol and antioxidant up to the final volume.

### Example 22 Preparation of sustained formulation containing 3% ivermectin

Take 9kg of solid dispersion particles (with a fineness below 300µm) of ivermectin / hydrogenated castor oil/PVP (1:0.7:0.3, W/W/W), add 30~50ml of dimeticone (20 mm²/S) grind with a colloid mill or a planetary mill so that the fineness of solid dispersion in the system falls between 70µm and 100 µm, then add dimeticone (20 mm²/S) containing trichloro-tert-butyl-alcohol and antioxidant up to the final volume.

### Example 23 Preparation of solid dispersion containing avermectin drugs

Take avermectin drugs and hydrogenated castor oil, and add in ethanol, acetone, ethyl acetate or other solvents with a low boiling point. Dissolve the avermectin drugs and melt the hydrogenated castor oil by heating the mixture, then cool under agitation. When the mixture has solidified, dry or reduce pressure and dry. With the solvent removed, solid dispersion will be obtained. Grind the solid dispersion and solid dispersion particles will be thus obtained.

### Example 24 Preparation of solid dispersion containing avermectin drugs

Take avermectin drugs and hydrogenated castor oil, add in ethanol, acetone, dimethyl acetoamide or dimethyl formamide. Dissolve the avermectin drugs and melt the hydrogenated castor oil by heating the mixture, then pour into cold water under agitation. When the mixture has solidified, have it filtered and get the solid material dried or dried by reducing pressure. Solid dispersion will thus be obtained. Grind the solid dispersion, and solid dispersion particles will be thus obtained.

### Example 25 Preparation of solid dispersion containing avermectin drugs

Take avermectin drugs, hydrogenated castor oil, and ethyl cellulose, add in acetone, dimethyl acetoamide or N-methyl pyrrolidone. Dissolve the avermectin drugs and ethyl cellulose and melt the hydrogenated castor oil by heating the mixture. Then pour the mixture into cold water under agitation. When the mixture has solidified, filter and get the solid material dried or dried by reducing pressure. Solid dispersion will thus be obtained. Grind the solid dispersion, and solid dispersion particles will be thus obtained.

### Example 26 Preparation of solid dispersion containing avermectin drugs

Take avermectin drugs, ethyl cellulose and hydrogenated castor oil, and add in acetone, ethyl acetate or other solvents with a low boiling point. Dissolve the avermectin drugs and ethyl cellulose and melt the hydrogenated castor oil by heating the mixture, then cool it down under agitation. When the mixture has solidified, have it dried or get dried by reducing pressure. With the solvent removed, solid dispersion will be obtained. Grind the solid dispersion, and solid dispersion particles will be thus obtained.

Example 27 Preparation of solid dispersion containing avermectin drugs

Take avermectin drugs, polyvinylpyrrolidone and hydrogenated castor oil, and add in ethanol. Dissolve the avermectin drugs and polyvinylpyrrolidone and melt the hydrogenated castor oil by heating the mixture, then cool under agitation. When the mixture has solidified, have it dried or get dried by reducing pressure. With the solvent removed, solid dispersion will be obtained. Grind the solid dispersion, and solid dispersion particles will be thus obtained.

In the given examples of the present invention, there is no description of aseptic operation and the fact that materials should be sterilised, which are well known by technicians in this field. This does not mean that no requirement of aseptic technique should be met when preparing the formulations described in the present invention.

## Claims

1. The preparation of sustained formulation using dimeticone as the carrier, with the formulation consisting of:
a. Therapeutic drug or active ingredient 0.5~40% (W/V);
b. Dimeticone up to 100% (V/V);
c. Other stabilisers, antioxidants, local analgesics and materials for sustained release may also be added to the formulation.

2. Formulations as described in Patent Claim 1, occurring in therapeutic drugs or active ingredients as follows:
(1) Avermectin antiparasitic drugs, including: abamectin, ivermectin, emamectin, eprinomectin, doramectin, moxidectin, 4"-deoxy-4"-methylamino-avermectin B₁ and other avermectin derivatives.
(2) Non-steroidal anti-inflammatory drugs (NSAIDs), including: salicylic acid derivatives, pyrazolones, para amino phenol derivatives, indole and indene acetic acid, arylkanoic acids (such as heteroaryl acetic acid and aryl propionic acid), 1, 2-benzothiazine (such as enolic acids ), alkanone, anthranalic acids (fenamic acids) and other COX-2 inhibitors; preferred NSAIDs used in the preparation of the formulation of this invention such as indomethacin, ketoprofen, meloxican, naproxen, caprofen, ketorolac, flunixin, diclofenac and piroxican.
(3) Other antiparasitic drugs, including imidacloprid, diflubenzuron, lufenuron, methoprene, fipronil, pyridinol, cyromazine, triazine anti-isospora drugs (toltrazuril, diclazuril), closantel or its sodium salt, albendazole sulphoxide or albendazole sulphoxide chloride. These antiparasitic drugs may be combined with avermectin antiparasitic drugs and prepared as a compound formulation.
(4) Antibiotics, including: cephalosporins, cillins, ESBL inhibitors, thiamulin base or thiamulin fumerate, tylosins, (tylosin, tilmicosin, isvaleryl tylosin tartrate), doxycycline or doxycycline hydrochloride, minocycline, gentamycin, lincomycin, clindamycin, neomycin, polymycin, quinolone antibiotics and sulphanilamide antibiotics. More than one of these can be used together in a formulation.
(5) Sex hormone agents, including oestrogen, progesterone, androgen.
(6) Oily soluble vitamins.
(7) Water-insoluble or water micro-soluble mineral elements.

3. In accordance with the formulation described in Patent Claim 1, characteristics present in the aforementioned stabilisers and materials for sustained release include: non-ionic surfactants, suspending agents, hydrophilic materials for sustained release and hydrophobic materials for sustained release.

4. In accordance with the formulation described in Patent Claim 3, characteristics present in the aforementioned non-ionic surfactants, suspending agents, hydrophilic materials for sustained release and hydrophobic materials for sustained release include: glycerine fatty acid esters, polyglycerol fatty acid esters, sucrose esters, sorbitan fatty acid esters (Span), polyoxyethylene sorbitan fatty acid ester condensate (Tween), polyoxyethylene fatty acid esters (Myrjs), polyoxyethylene fatty acid alcohol condensate (Brijs), Pingpingjia (Paregal), emulsifier (OP), polyoxyethylene castor oil condensate, polyoxyethylene hydrogenated castor oil condensate, Pluronic, fatty acid esters at a solid state under 40°C (such as glycerine monostearate, hydrogenated castor oil etc), lanolin, cetyl alcohol, stearic acid, polyvinylpyrrolidone (PVP), polyethylene glycol with a molecular weight greater than 1000, gelatine, Arabic gum, ethyl cellulose, poly resin and polyethylene butyral. Preferred stabilisers and materials for sustained release are: Tween, Span, ethyl cellulose, hydrogenated castor oil, aluminium stearate, polyvinylpyrrolidone (PVP) and polyethylene glycol with a molecular weight greater than 1000. Two or more of these may be used in combination.

5. In accordance with the formulation described in Patent Claims 1-4, ingredients present in the formulations are:
a. Avermectin drug 0.5~30% (WN);
b. Hydrogenated castor oil 0~10% (W/V);
c. Local analgesics 0.5~2.5% (WN);
d. BHT or BHA or PG or a combination 0.2% (WN);
e. Dimeticone up to 100% (V/V).

6. In accordance with that described in Patent Claim 5, preferred ingredients present in the formulations are:
a. Avermectin drug 1~10% (W/V);
b. Hydrogenated castor oil 1~5% (WN);
c. Trichloro-tert-butyl-alcohol 0.5 (WN);
d. BHT/BHA/PG 0.2% (WN);
e. Dimeticone up to 100% with a viscosity of less than 100mm²/S (VN).

7. In accordance with the formulation described in Patent Claim 6, preparation methods of the aforementioned formulations are:
Method (1): Take the avermectin drug, add 2~5 times the weight of alcohol, acetone or other organic solvent with a low boiling point, add hydrogenated castor oil (optional), and dissolve/fuse at approximately 85°C. Under agitation, cool to below 30°C, then add dimeticone and an auxiliary agent up to the final volume. Reduce pressure and remove the alcohol, acetone or other organic solvent with a low boiling point and homogenise (for example, through a colloid mill or a 100~200 mu sieve).
Method (2): Take the avermectin drug and hydrogenated castor oil, add a small quantity of dimeticone and melt the hydrogenated castor oil at a temperature of approximately 90°C. Then cool under agitation to form a fatty or relatively viscous and dense liquid and grind (for example, through a colloid mill or a planetary mill). When the fineness of the avermectin drug reaches below 100 µm, add a residual medium or auxiliary agent up to the final volume.
Method (3): Scatter the avermectin drug which has already been micronised (a fineness of less than 100 µm) into hydrogenated castor oil which has already been melted. Then add a small quantity of dimeticone, homogenise, and add a residual medium and auxiliary agent up to the final volume. Alternatively, heat the avermectin drug which has already been micronised (a fineness of less than 100 µm), hydrogenated castor oil and an appropriate amount of dimeticone. Melt the hydrogenated castor oil and, under agitation, cool to below 30°C. Homogenise, and then add a residual medium and auxiliary agent up to the final volume.
Method (4): Mix a solid dispersion containing the avermectin drug and hydrogenated castor oil with dimeticone. Heat to approximately 90°C under agitation and, once melted, agitate and cool. Homogenise (for example, through a colloid mill or a 100~200 mu sieve) and add a residual medium and auxiliary agent up to the final volume.
Method (5): Scatter a solid dispersion containing the avermectin drug and hydrogenated castor oil in dimeticone and grind (for example, through a colloid mill or a planetary mill). When the fineness of the solid dispersion reaches below 150 µm, add dimeticone and auxiliary agent up to the final volume. Alternatively, scatter a solid dispersion (fineness less than 150 µm) containing the avermectin drug and hydrogenated castor oil in dimeticone, homogenise, and add dimeticone and a residual agent up to the final volume.

8. In accordance with that described in Patent Claims 1-4, preparation methods and composition of the formulation are:
(1). Composition of formulation:
a. Drug carrier micro particles containing avermectin drug 2~35% (WN);
b. Suspending agent (such s hydrogenated castor oil) 0~3% (WN);
c. Local analgesic 0.5~2.5% (WN);
d. Dimeticone up to 100% (V/V).
(2). Preparation method:
Scatter the drug carrier micro particles containing the avermectin drug (fineness less than 360 µm) in dimeticone and grind (using a planetary mill or colloid mill). When the drug carrier micro particles are less than 150 µm, add dimeticone which may or may not include a suspending agent, up to the final volume. Mix. Alternatively, scatter the drug carrier micro particles containing the avermectin drug (fineness less than 120 µm) in dimeticone which may or may not include a suspending agent, and homogenise.

9. In accordance with that described in Patent Claim 8, the aforementioned drug carrier micro particle containing the avermectin drug is a solid dispersion composed of avermectin antiparasitic drugs and materials for sustained release or another kind of drug carrier micro particle (such as microspheres, micro-capsules, nano particles or liposomes).

10. In accordance with that described in Patent Claim 9, the aforementioned materials for sustained release include hydrophilic carrier materials and hydrophobic carrier materials; the preferred hydrophilic carrier materials are gelatine, Arabic gum, polyethylene glycol with a molecular weight greater than 1000 and polyvinylpyrrolidone (PVP); preferred hydrophobic carrier materials are ethyl cellulose, polyethylene butyral and hydrogenated vegetable oil (such as hydrogenated castor oil); especially preferred carrier materials are ethyl cellulose, hydrogenated castor oil, polyethylene glycol with a molecular weight greater than 1000 and polyvinylpyrrolidone (PVP). More than one kind may be used together.

11. In accordance with that described in Patent Claims 6-8, the aforementioned formulation is used in the prevention and treatment of parasitic diseases in animals, is injectable, preferably by means of a subcutaneous, and particularly by injection behind the ear, in the neck or in the back of the animal. The dosage is 0.2~6mg active ingredient/kilogramme of body weight (kg b.w), with an appropriate dosage being 1~3mg active ingredient/kg b.w).

12. In accordance with that described in Patent Claims 1~4, composition of the formulation is:
a. Non-steroidal anti-inflammatory drugs (NSAIDs) 1~15% (W/V);
b. Hydrogenated castor oil 0~5% (W/V);
c. Dimeticone up to 100% (V/V);
d. An antioxidant or local analgesic may also be added.

13. In accordance with the formulation described in Patent Claim 12, preparation methods of the aforementioned formulation are:
Method (1): Take the NSAIDs, add 2-5 times the weight of alcohol, acetone or other organic solvent with a low boiling point, hydrogenated castor oil (optional), and dissolve/fuse at approximately 85°C. Under agitation, cool, and then add dimeticone up to the final volume. Reduce pressure and remove the alcohol, acetone or other organic solvent with a low boiling point and homogenise (for example, through a colloid mill or a 100~200 mu sieve).
Method (2): Take the NSAIDs and hydrogenated castor oil, add a small quantity of dimeticone and melt the hydrogenated castor oil at a temperature of approximately 90°C. Then cool under agitation to form a fatty or relatively viscous and dense liquid and grind (for example, through a colloid mill or a planetary mill). When the fineness of the NSAIDs reaches below 100 µm, add a residual medium and auxiliary agent up to the final volume.
Method (3): Scatter the NSAIDs which have already been micronised (a fineness of less than 100 µm) into hydrogenated castor oil which has already been melted. Then add dimeticone, homogenise, and add a residual medium and auxiliary agent up to the final volume.
Method (4): Mix a solid dispersion containing the NSAIDs and hydrogenated castor oil with dimeticone. Heat to a temperature of approximately 90°C under agitation and wait for it to melt. Then cool and continue agitation until homogenisation, and add a residual medium and auxiliary agent up to the final volume.
Method (5): Scatter a solid dispersion containing the NSAIDs and hydrogenated castor oil in dimeticone and grind (for example, through a colloid mill or a planetary mill). Homogenise, and when the fineness of the solid dispersion is less than 120 µm, add dimeticone and auxiliary agent up to the final volume.

14. In accordance with that described in Patent Claims 1~4, preparation methods and composition of the formulation are:
(1) Composition of formulation:
a. Fipronil, diflubenzuron or imidacloprid 2~10% (W/V);
b. Hydrogenated castor oil 0.2~5% (W/V);
c. Dimeticone up to 100% (V/V);
d. An antioxidant or local analgesic may also be added.
(2) Preparation methods:
Method (a): Take the fipronil or imidacloprid, add alcohol, acetone or other organic solvent with a low boiling point, add hydrogenated castor oil, and dissolve/fuse at approximately 85°C. Under agitation, cool, and then add dimeticone and an auxiliary agent up to the final volume. Reduce pressure and remove the alcohol, acetone or other organic solvent with a low boiling point and homogenise (for example, through a colloid mill).
Method (b): Take the fipronil, diflubenzuron or imidacloprid and hydrogenated castor oil, add a small quantity of dimeticone and melt the hydrogenated castor oil at a temperature of approximately 90°C. Then cool under agitation to form a fatty or relatively viscous and dense liquid and grind (for example, through a colloid mill or a planetary mill). When the fineness of the fipronil, diflubenzuron or imidacloprid reaches below 100 µm, add a residual medium and auxiliary agent up to the final volume.
Method (c): Scatter the fipronil, diflubenzuron or imidacloprid which has already been micronised (a fineness of less than 100 µm) into hydrogenated castor oil which has already been melted. Then add dimeticone, homogenise, and add a residual medium and auxiliary agent up to the final volume.
Method (d): Mix a solid dispersion containing fipronil, diflubenzuron or imidacloprid and hydrogenated castor oil with dimeticone. Heat to approximately 90°C under agitation and wait for it to melt. Then cool and continue agitation until homogenisation, and add a residual medium and auxiliary agent up to the final volume.
Method (e): Scatter a solid dispersion containing fipronil, diflubenzuron or imidacloprid and hydrogenated castor oil in dimeticone and grind (for example, through a colloid mill or a planetary mill). When the fineness of the solid dispersion is less than 120 µm, add dimeticone and auxiliary agent up to the final volume.

15. In accordance with that described in Patent Claims 1-4, preparation methods and composition of the formulation are:
(1) Composition of formulation:
a. Penicillin or cephalosporin drugs 2~40% (WN);
b. Hydrogenated castor oil 0~5% (WN);
c. Dimeticone up to 100% (V/V);
d. An antioxidant or local analgesic may also be added.
(2) Preparation methods:
Method (a): Take the penicillin or cephalosporin drugs and add a small quantity of dimeticone to form a viscous and dense liquid. This may or may not then be ground (for example, through a colloid mill or a planetary mill). Under agitation, add dimeticone containing melted hydrogenated castor oil and cool under agitation to form a fatty or relatively viscous and dense liquid. Grind (for example, through a colloid mill or a planetary mill ) and when the fineness of the penicillin or cephalosporin drugs reaches below 100 µm, add a residual medium and auxiliary agent up to the final volume.
